(19) Europäisches Patentamt
European Patent Office
Office européen des brevets

(11) EP 4 206 256 A1

(12) **EUROPEAN PATENT APPLICATION**
published in accordance with Art. 153(4) EPC

(43) Date of publication:
**05.07.2023 Bulletin 2023/27**

(21) Application number: **21957733.5**

(22) Date of filing: **19.10.2021**

(51) International Patent Classification (IPC):
***C08G 61/12*** *(2006.01)*

(52) Cooperative Patent Classification (CPC):
**C08G 61/12; H10K 10/40; H10K 30/50; H10K 50/10; H10K 85/10;** Y02E 10/549

(86) International application number:
**PCT/CN2021/124545**

(87) International publication number:
**WO 2023/056662 (13.04.2023 Gazette 2023/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.10.2021 CN 202111177652**

(71) Applicant: **South China University of Technology Guangdong 510641 (CN)**

(72) Inventors:
- **HUANG, Fei**
  **Guangzhou**
  **Guangdong 510641 (CN)**
- **TANG, Haoran**
  **Guangzhou**
  **Guangdong 510641 (CN)**

(74) Representative: **Cueto, Sénida**
**SP3 Patents S.L.**
**Los Madroños, 23**
**28891 Velilla de San Antonio (ES)**

# (54) N-TYPE CONJUGATED POLYMER BLEND, AND PREPARATION METHOD THEREFOR AND USE THEREOF

(57) The present invention relates to an n-type conjugated polymer blend, which is made from aromatic diketone with active methylene or an enolic transformation product thereof, and is obtained directly by polymerization reaction in the presence of an oxidant. The reaction described does not require precious metal catalysis and is insensitive to a reaction atmosphere. A process is simple and inexpensive and suitable for commercial applications. Meanwhile, the modulation of the conductivity of this n-type conjugated polymer can be achieved by ionic modification. This n-type conjugated polymer can be applied to an organic optoelectronic device to achieve an excellent photovoltaic effect.

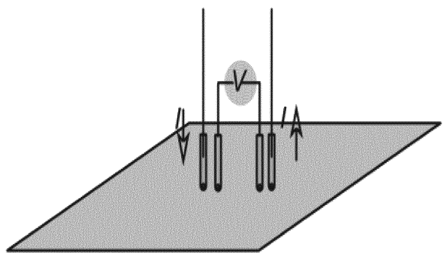


Fig. 4

EP 4 206 256 A1

## Description

### TECHNOLOGY FIELD

The present invention belongs to the technical field of organic semiconductor materials, and specifically relates to an n-type conjugated polymer blend, a method for preparing the same and application.

### BACKGROUND

The structure of a conjugated polymer contains a conjugated system consisting of an off-domain $\pi$-electron and thus exhibits unique properties of a semiconductor or a conductor. Since the advent of conductive polyacetylene has allowed the boundary between the insulating and conducting properties of a plastic to be broken, an organic conjugated polymer has shown the prospect for a wide range of applications in an electronic device. At present, the conjugated polymer has achieved a series of impressive results in an organic solar cell (OSC), an organic light emitting diodes (OLED), an organic field effect transistor (OFET), and organic thermoelectric (OTE).

High conductivity, as an important indicator of the performance of an organic conductive polymer, has been the subject of extensive research by scientists. In the quest to obtain highly conductive organic materials, a variety of structures have been developed, such as polyaniline, polythiophene, and polypyrrole. One of the most representative materials, that is, poly 3,4-ethylenedioxythiophene: polystyrene sulfonate acid (PEDOT:PSS), as one of the materials that have been commercially applied, has achieved great success in the electronic device due to high conductivity and printable processing, with an annual output value of more than 10 billion yuan.

Although very great progress has been made in the conjugated polymer, the current high-performance conductive materials all exhibit p-type transport properties, and the performance of an n-type conductive material still lags far behind, especially an organic n-type conjugated polymer with high conductivity, which lacks a corresponding synthetic strategy. The development of an n-type organic semiconductor material with high conductivity, simple synthesis, low costs, and solution processability is currently a hot research topic due to the limitation of the low electron mobility, poor air stability, and the need for a long insulating alkyl chain of the n-type organic semiconductor material for solution processing.

The literature (Toward High Performance n-Type Thermoelectric Materials by Rational Modification of BDPPV Backbones. J. Am. Chem. Soc. 2015, 137, 6979) reported a benzodifurandione-based polymer BDPPV, which was optimized with a dopant to achieve conductivity up to 14 S/cm. The literature (High Conductivity and Electron-Transfer Validation in an n-Type Fluoride-Anion-Doped Polymer for Thermoelectrics in Air. Adv. Mater. 2017, 29, 1606928) reported the use of tetrabutylammonium fluoride doped with a benzodifurandione -based polymer to obtain an n-type thermoelectric material with good air stability. In addition, the literature (Rigid Coplanar Polymers for Stable n-Type Polymer Thermoelectrics. Angew. Chem. Int. Ed. 2019, 58, 11390) reported the use of condensation reaction and polymerization of acid catalyzed aldol to obtain a polymer LPPV with air stability and high conductivity while avoiding the use of a precious metal catalyst. The literature (A thermally activated and highly miscible dopant for n-type organic thermoelectrics. Nat. Commun. 2020, 11, 3292.) reported the enhancement of the conductivity of the n-type conjugated polymer by constructing a new dopant to improve the dopant-polymer backbone compatibility, but the highest value still did not exceed 50 S/cm. The literature (Persistent conjugated backbone and disordered lamellar packing impart polymers with efficient n-doping and high conductivities. Adv. Mater. 2021, 33, 200546. 33, 2005946.) reported that the n-type conjugated polymer with conductivity close to 100 S/cm were obtained by synergistic modulation of polymer planarity and dopant compatibility. At present, although the conductivity of the n-type conjugated polymer is further improved by the adjustment of the polymer and the dopant, there is still a big gap compared with the performance of a p-type conjugated polymer over 1000 S/cm. Most of n-type conductive conjugated polymers are currently prepared by first constructing a polymer backbone and then doping with an additional dopant (e.g., N-DMBI) to obtain high conductivity, which is a long, unstable and costly process.

In summary, there is an urgent need to develop an n-type highly conductive conjugated polymer with a simple structure, easy synthesis, low costs and solution processability, to synthesize the n-type highly conductive conjugated polymer directly by an applicable commercial preparation method, to apply the n-type highly conductive conjugated polymer in an organic photovoltaic field, and to achieve the desired photovoltaic effect.

### SUMMARY

To overcome the shortcomings and deficiencies of the prior art, an objective of the present invention is to provide an n-type conjugated polymer containing a counterion modified by different counterions and a method for preparing the same. The raw materials used in the method of the present invention are aromatic diketones with an active methylene, and polymerized in the presence of an oxidant and/or an ion-exchange adjuvant to directly obtain the n-type

conjugated polymers containing the counterion. The described reaction does not require precious metal catalysis, is insensitive to a reaction atmosphere and is a simple and inexpensive process suitable for a commercial application. The n-type conjugated polymer containing the counterion obtained by the method of the present invention has good solubility in a general organic solvent and can be processed in solution. Meanwhile, this n-type conjugated polymer containing the counterion can be applied to an organic optoelectronic device to achieve excellent optoelectronic effects.

The term "aromatic ring" of the present invention refers to a ring-like structure possessing a conjugated planar ring system in which an interatomic bonding is covered by an off-domain π-electron cloud, such as benzene ring and derivatives thereof.

The term "aromatic heterocycle ring" of the present invention refers to a ring structure that possesses a conjugated planar ring system in which an interatomic bonding is covered by the off-domain π-electron cloud, and in which atoms forming the ring contain at least one heteroatom (e.g., N, O, S, etc.) in addition to a carbon atom, such as thiophene, furan, and pyrrole.

The term "fused aromatic ring" of the present invention refers to a structure that has a conjugated planar ring system in which an interatomic bonding is fused by two or more aromatic rings (i.e., share ring edges) covered by the off-domain π-electron cloud, such as naphthalene, anthracene and derivatives thereof.

The term "fused aromatic heterocycle ring" of the present invention refers to a structure having a conjugated planar ring system in which an interatomic bonding is covered by the off-domain π-electron cloud and in which at least one ring contains at least one heteroatom (e.g. N, O, and S) in addition to the carbon atom, such as quinoline, indole and derivatives thereof.

The objective of the present invention is achieved by the following technical solutions: An n-type conjugated polymer blend includes an n-type conjugated polymer containing a counterion, and/or an n-type conjugated polymer. wherein,

the n-type conjugated polymer containing the counterion includes one or more polymerization units 1, the polymerization unit 1 has the following structure (I):

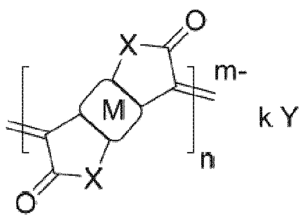

(I);

the n-type conjugated polymer includes one or more polymerization units 2, the polymerization unit 2 has the following structure (II):

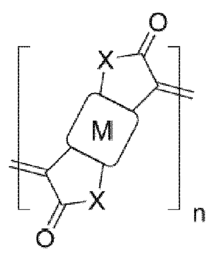

(II);

in each of the polymerization unit 1 or the polymerization unit 2,

X is independently selected from O, S, Se, Te or N-$R_1$;

the $R_1$ is selected from one or more of a hydrogen atom, alkyl, alkylidene, an alkyl derivative, and an alkylidene derivative;

one or more carbons on the alkyl derivative or the alkylidene derivative are substituted with one or more of an oxygen atom, amino, sulfonyl, carbonyl, aryl, alkenyl, alkynyl, ester, cyano, and nitro;

and/or

one or more hydrogens on the alkyl derivative or the alkylidene derivative are substituted with one or more of halogen, hydroxyl, the amino, carboxyl, the cyano, the nitro, the aryl, alkylene, and alkyne;

where m, n and k are positive integers;
the M is a conjugated portion of a structure of the n-type conjugated polymer containing
the counterion or the n-type conjugated polymer, and a structure of the M is selected from one of aromatic ring, aromatic heterocycle, fused aromatic ring, fused aromatic heterocycle;
the Y is a counterion in the structure of the n-type conjugated polymer containing the counterion or the n-type conjugated polymer, and the counterion is selected from one of an organic cation or an inorganic cation;
the n-type conjugated polymer blend is prepared from a raw material (III), and the raw material (III) has the following structure:

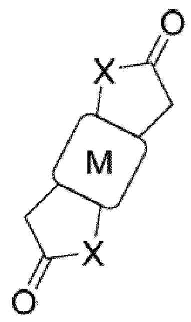

and/or an enol-transformed form of the raw material; the enol-transformed form is

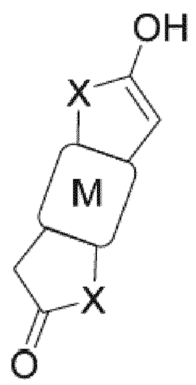

and/or

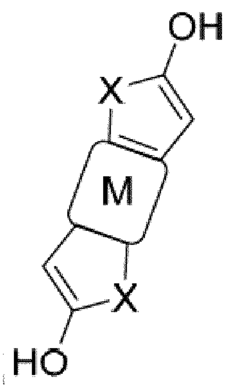

.

The enol-transformed form described is the result of the interaction between the raw material (III) and other substances (such as a polar solvent, an acid or a base).

In the technical solution of the present invention, a product prepared from the raw material (III) is a blend containing both an n-type conjugated polymer in an ionic state (with a structural formula (I)) and a neutral n-type conjugated polymer (with a structural formula (II)). A dynamic interconversion is provided between the n-type conjugated polymer containing the counterion and the n-type conjugated polymer. This is mainly due to the following resonance transition in the n-type conjugated polymer blend when free positive hydrogen ions are present in a system.

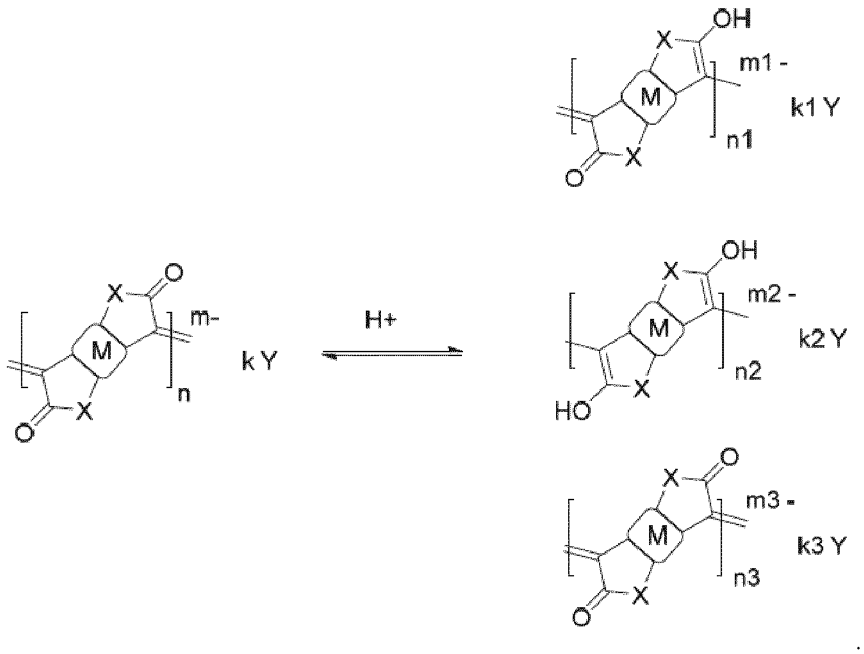


Further, to illustrate when Y is a hydrogen positive ion, the system containing the n-type conjugated polymer in the ionic state can be transformed into a blend of the following resonance, i.e., the dynamic interconversion is provided between the n-type conjugated polymer containing the counterion and the n-type conjugated polymer, with a ratio of the amount of substance of the n-type conjugated polymer containing the counterion to the amount of substance of n-type conjugated polymer being 0.1-10.

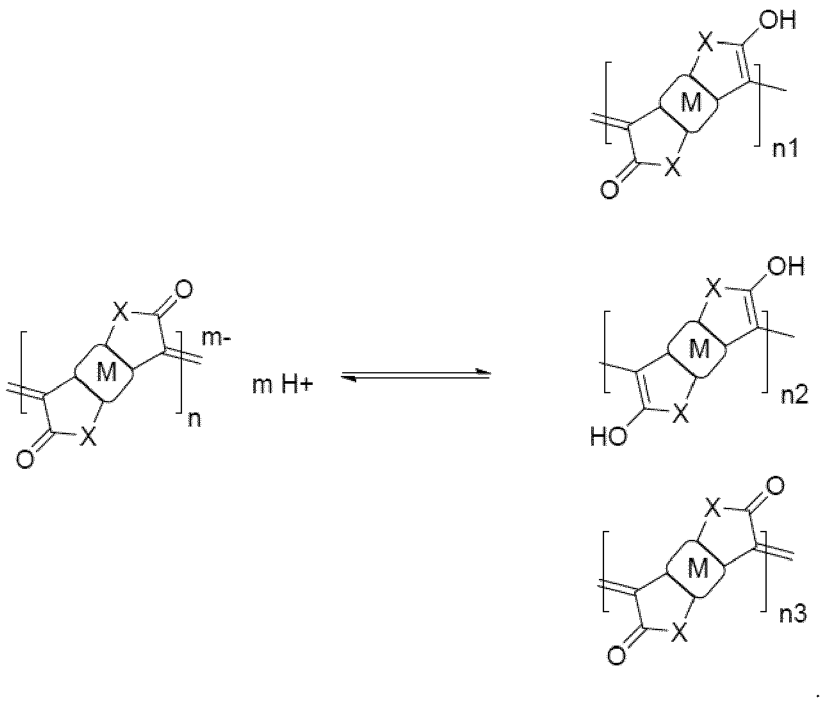

Further, the n-type conjugated polymer containing the counterion and the n-type conjugated polymer can further be self-assembled by interaction into the following supramolecule. A supramolecular structure can facilitate intra/intermolecular charge transfer to form the n-type conjugated polymer blend with ultra-high conductivity.

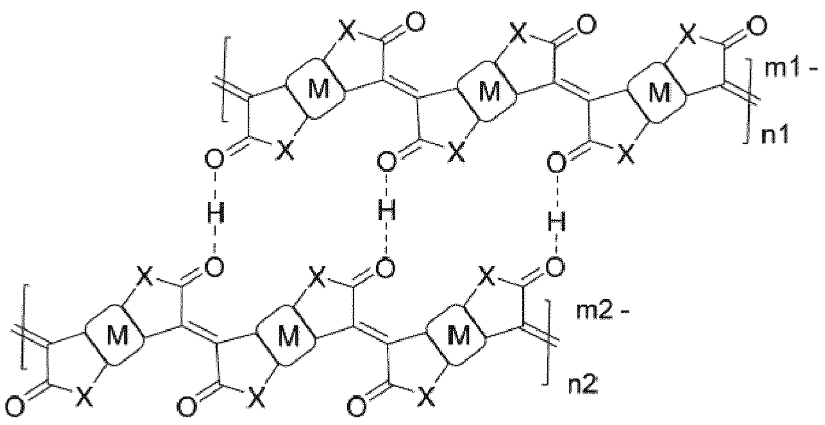

Further, the n-type conjugated polymer containing the counterion has multiple resonance forms. As an example, when the M is a benzene ring structure and the number of negative charges in a backbone is 1 (m=1), the following resonance forms are included but not limited to:

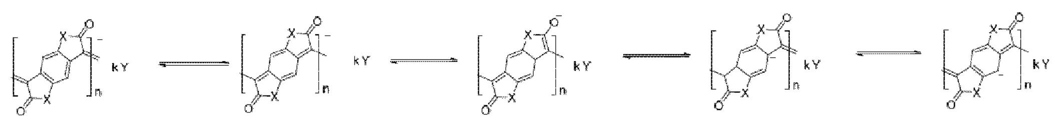

For convenience, the contents of the present invention are expressed in a first resonance form.

Further, a structure of a conjugated portion M of a structure of the n-type conjugated polymer containing the counterion is selected from the following structures:

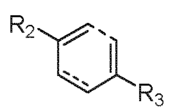
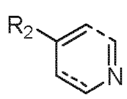
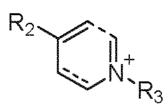
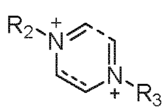
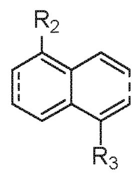
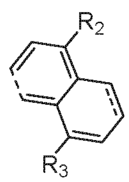
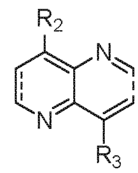
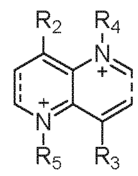
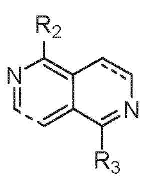
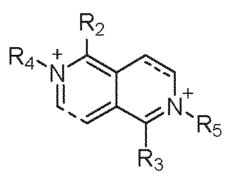
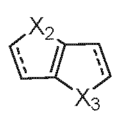
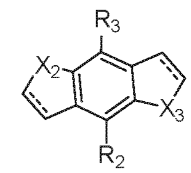
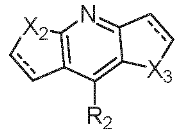
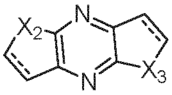
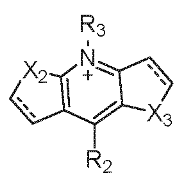
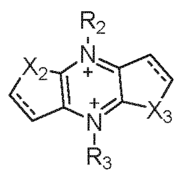

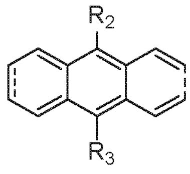 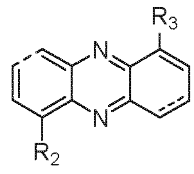 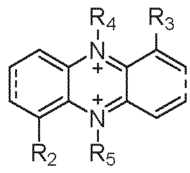 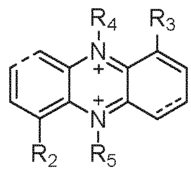

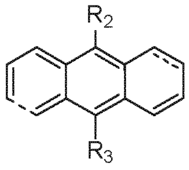 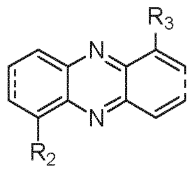 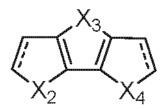 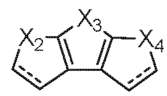

;

wherein the $X_2$-$X_4$ are independently selected from O, S, Se, Te or N-$R_6$;

$R_2$-$R_6$ is independently selected from one or more of the hydrogen atom, the hydroxyl, the nitro, the halogen, the cyano, the nitro, the alkyl, the alkyl derivative, the alkylidene, and the alkylidene derivative;

One or more carbons on the alkyl derivative or the alkylidene derivative are substituted with one or more of the oxygen atom, the amino, the sulfonyl, the carbonyl, the aryl, the alkenyl, the alkynyl, the ester, the cyano, and the nitro; and/or

One or more hydrogens on the alkyl derivative or the alkylidene derivative are substituted with one or more of the halogen, the hydroxyl, the amino, the carboxyl, the cyano, the nitro, the aryl, the alkylene, and the alkyne.

The dashed line (---) in the aromatic ring in an optional object of the structure of M above indicates the mutual fusing of an adjacent five-membered ring or four-membered ring here,

i.e., a shared ring edge. For example, when the structure of M is expressed as 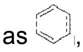, the actual structure of the polymerization unit of the n-type conjugated polymer containing the counterion represented by the M is:

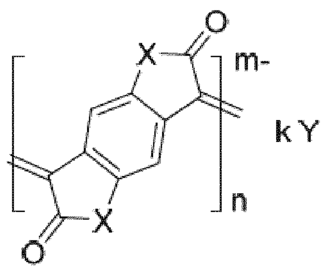

Further, the structure of the counterion portion Y of the structure of the n-type conjugated polymer is selected from one of the following structures:

$H^+$ 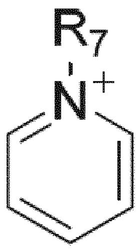 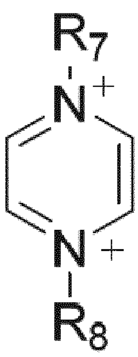 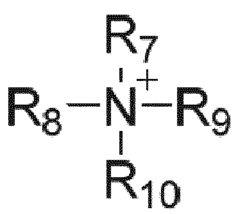 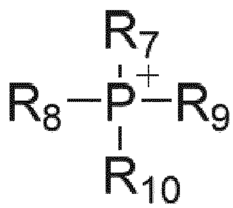

$Na^+$, $K^+$, $Mg^{2+}$, $Ca^{2+}$, $Ba^{2+}$, $Al^{3+}$, $Fe^{2+}$, $Fe^{3+}$, $Zn^{2+}$, $Cu^{2+}$ and $Ag^+$;

or is selected from one of an amine-salt-type cationic surfactant, a quaternary-ammonium-salt-type cationic surfactant, a heterocycl-type cationic surfactant, a sulfonium salt, iodine, and other salt compounds.

Further, (1) when the n-type conjugated polymer containing the counterion is a homopolymer, the structure of the n-type conjugated polymer containing the counterion is selected from one of the following structures:

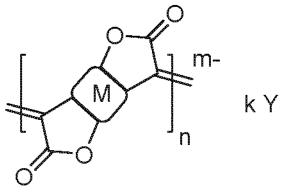
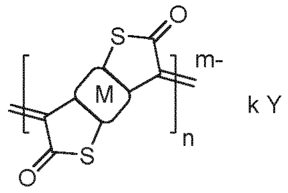
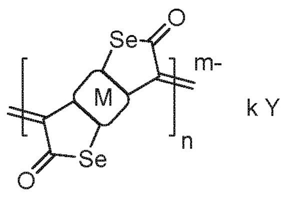
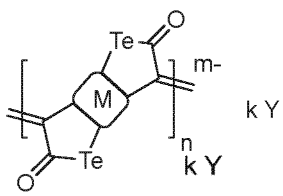
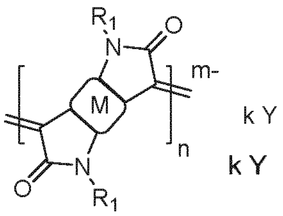

;

or

(2) when the n-type conjugated polymer containing the counterion is a copolymer,

the structure of each of the polymerization unit 1 of the n-type conjugated polymer containing the counterion is independently selected from the following structures:

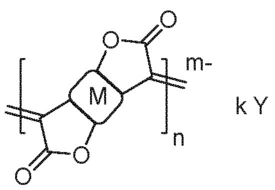
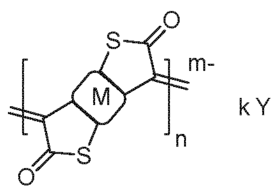
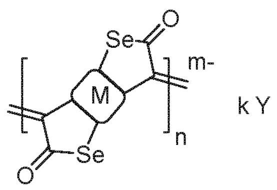
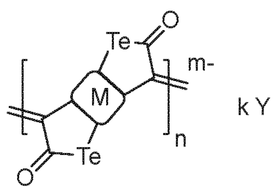
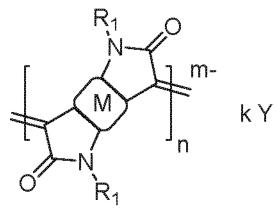

;

wherein,

the $R_1$ is selected from one or more of a hydrogen atom, alkyl, alkylidene, an alkyl derivative, and an alkylidene derivative;

One or more carbons on the alkyl derivative or the alkylidene derivative are substituted with one or more of the oxygen atom, the amino, the sulfonyl, the carbonyl, the aryl, the alkenyl, the alkynyl, the ester, the cyano, and the nitro;

and/or

One or more hydrogens on the alkyl derivative or the alkylidene derivative are substituted with one or more of the halogen, the hydroxyl, the amino, the carboxyl, the cyano, the nitro, the aryl, the alkylene, and the alkyne.

where m, n and k are positive integers;

the M is the conjugated portion of the structure of the n-type conjugated polymer containing the counterion, and the structure of the M is selected from one of an the aromatic ring, the aromatic heterocycle, the fused aromatic ring, and the fused aromatic heterocycle;

the Y is the counterion portion of the n-type conjugated polymer containing the counterion, and the counterion

is selected from one of an organic cation and an inorganic cation. Further, (1) when the n-type conjugated polymer is the homopolymer, the structure of the n-type conjugated polymer is selected from one of the following structures:

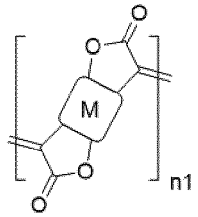 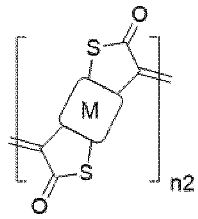 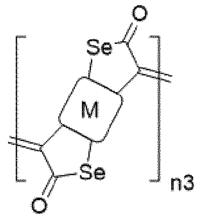

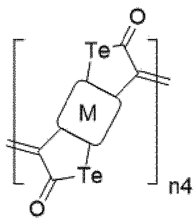 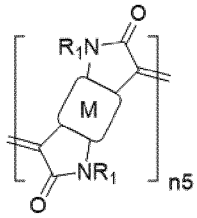

;

(2) when the n-type conjugated polymer is the copolymer,
a structure of each of the polymerization unit 2 of the n-type conjugated polymer is independently selected from the following structures:

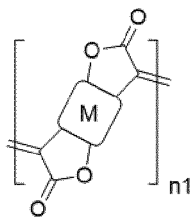 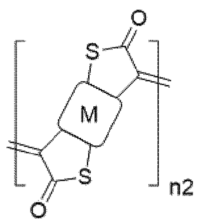 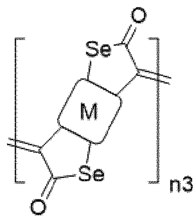

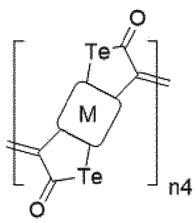 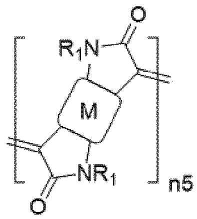

;

the n1-n5 are independently positive integers.

Another objective of the present invention is to provide a method for preparing the above-mentioned n-type conjugated polymer containing a counterion:

(1) when the n-type conjugated polymer containing the counterion and/or the n-type conjugated polymer is a homopolymer, a method of preparing an n-type conjugated polymer blend includes the following steps of:
mixing a raw material (III) and an oxidant and/or an ion exchange adjuvant in a solvent and heating to obtain a homopolymer;
or
(2) when the n-type conjugated polymer containing the counterion and/or the n-type conjugated polymer is a copolymer, the method of preparing the n-type conjugated polymer blend includes the following steps of:
mixing two or more of the raw materials (III), and the oxidant and/or the ion exchange adjuvant in the solvent and heating to obtain the copolymer;

The method for preparing the described n-type conjugated polymer containing the counterion and/or the n-type conjugated polymer can be achieved either by direct addition of an ion-exchange additive to a reaction substance as described above, or by first preparing one of the n-type conjugated polymer containing the counterion and/or the n-type conjugated polymer and then obtaining the other n-type conjugated polymer containing the counterion and/or the n-type conjugated polymer in the form of post-ion exchange.

Further, the oxidant is selected from one or more of an organic oxidant and an inorganic oxidant.

Further, the oxidant is selected from one or more of oxygen, peroxide, metal halide, persulfate, perborate, subhaloate, halogenite, a quinones compound, and a benzylhydroperoxide compound.

Specifically, the above oxidants may be, but not limited to, one of the oxygen, hydrogen peroxide, sodium peroxide, potassium peroxide, calcium peroxide, zinc peroxide, copper peroxide, ferric nitrate, zinc nitrate, nickel nitrate, aluminum nitrate, magnesium nitrate, ammonium nitrate, ferric fluoride, ferric chloride, ferric bromide, ferric iodide, sodium perchlorate, potassium perchlorate, sodium perbromide, potassium perbromide, sodium periodate, potassium periodate, potassium perchlorate, sodium perchlorate, potassium perbromide, sodium perbromide, magnesium perchlorate, sodium persulfate, potassium persulfate, magnesium persulfate, zinc persulfate, ferric persulfate, copper persulfate, calcium persulfate, potassium perborate, zinc perborate, magnesium perborate, calcium perborate, sodium hypofluorate, potassium hypofluorate, sodium hypochlorite, potassium hypochlorite, ferric hypochlorite, copper hypochlorite, sodium hypobromite, potassium hypobromite, sodium hypoiodite, potassium hypoiodite, sodium chlorite, potassium chlorite, ferric chlorite, sodium bromite, potassium bromite, sodium iodite, potassium iodite, benzoquinone and derivatives thereof, naphthoquinone and derivatives thereof, anthraquinone and derivatives thereof, phenanthrenequinone and derivatives thereof, benzylhydroperoxide and derivatives thereof; and a mixture of two or more of any of the above substances mixed in any ratio.

Further, the solvent is selected from a solvent 1 or a solvent 2, or a mixture of a solvent 1 and a solvent 2.

The solvent 1 is selected from a mixture of one or more of water, a nitrile solvent, an aromatic solvent, an alicyclic hydrocarbon solvent, an alicyclic hydrocarbon solvent, a halogenated hydrocarbon solvent, an alcohol solvent, an ether solvent, an ester solvent, a sulfone solvent, a ketone solvent, an amide solvent.
the solvent 2 is a deuterated solvent of the solvent 1.

Specifically, the above solvent 1, preferably a polar solvent common in the art, may be, but not limited to, one of tetrahydrofuran, methyltetrahydrofuran, methylene chloride, chloroform, ethyl acetate, propyl acetate, butyl acetate, ethyl propionate, propyl propionate, butyl propionate, methyl methacrylate, ethyl methacrylate, propyl methacrylate, toluene, xylene, trimethylbenzene, chlorobenzene, dichlorobenzene, trichlorobenzene, methanol ethanol, ethanol, propanol, ethylene glycol, isobutanol, propylene glycol, acetonitrile, formic acid, acetic acid, propionic acid, trifluoroacetic acid, dimethylformamide, dimethylacetamide, dimethyl sulfoxide, dimethylacetamide, acetone, butanone, cyclohexanone, methyl butanone, methyl ether, ethyl ether, propyl ether, pyridine, phenol, N-methylpyrrolidone, ethylene glycol monomethyl ether, triethylene glycol monomethyl ether, triethylamine, tetramethylethylenediamine, trioctylamine, aniline, and hexamethylphosphoramide, and a mixture of two or more of any of the above substances mixed in any ratio.

Specifically, the above solvent 2 is a deuterated solvent corresponding to the above solvent 1. For example, the solvent 2 may be, but not limited to, deuterated chloroform, deuterated chlorobenzene, deuterated ethanol, etc.

Preferably, the solvent is selected from the solvent 1.

For convenience, the contents of the present invention are expressed in terms of a blend containing the n-type conjugated polymer in an ionic state (having structural formula (I)), and a neutral n-type conjugated polymer (having structural formula (II)).

Another objective of the present invention is to provide application of the above-mentioned n-type conjugated polymer blend containing the counterion in an organic optoelectronic device.

The beneficial effects of the present invention are as follows:

1. The raw material of the n-type conjugated polymer blend obtained by the present invention is an aromatic diketone with an active methylene, which is obtained directly by polymerization reaction in the presence of an oxidant. The reaction does not require precious metal catalysis and is insensitive to a reaction atmosphere, which makes a process simple and inexpensive for large-scale commercial applications. The resulting product has excellent solubility and is suitable for use in a solution-processed organic optoelectronic device.
2. The n-type conjugated polymer blend obtained by the present invention can be self-assembled by in situ reaction and resonance transformation to form an n-type conductive material with strong aggregation and conductivity up to more than 2000 S/cm.
3. The resulting n-type conjugated polymer blend of the present invention can be used as a thermoelectric material to obtain a power factor close to 100 $\mu Wm^{-1}K^{-2}$ in an air environment without the use of an additional dopant.
4. The resulting n-type conjugated polymer blend of the present invention allows for systematic regulation of the n-type conjugated polymer in terms of thermoelectric power by the regulation of different counterions.

## BRIEF DESCRIPTION OF THE DRAWINGS

Fig. 1 shows an absorption spectrogram of a product in Embodiment 1 in a thin film state.
Fig. 2 shows a graph of a gel permeation chromatography test of a product in Embodiment 1.
Fig. 3 shows an X-ray photoelectron energy spectrum of a product in Embodiment 1, Fig. 3 (a) shows an XPS spectrum of an element of oxygen (oxygen 1s peak) and Fig. 3 (b) shows a total spectrum.
Fig. 4 shows a schematic diagram of a four-probe conductivity test of a product in Test Example 1.
Fig. 5 shows a graph of voltage versus current data for a four-probe conductivity test of a product in Embodiment 1 of Test Example 1, Fig. 5 (a) shows a graph of current measurements in a four-probe, and Fig. 5 (b) shows a graph of voltage measurements in a four-probe.
Fig. 6 shows a graph of a Seebeck coefficient test for a product in Embodiment 1.
Fig. 7 shows a schematic diagram of an organic electrochemical transistor device.
Fig. 8 shows a trans-conductivity test diagram of a product in Embodiment 1 in an organic electrochemical transistor device.

## DETAILED DESCRIPTION OF EMBODIMENTS

In order to illustrate the technical solutions of the present invention more clearly, the following embodiments are given. Unless otherwise stated, raw materials, reactions and post-processing means in the embodiments are common raw materials on market and technical means well known to a person skilled in the art.

As one of the raw materials in this embodiment of the invention, 3,7-dihydrobenzo[1,2-b:4,5-b'] difuran-2,6-dione, was prepared according to the literature (A BDOPV-Based Donor-Acceptor Polymer for High-Performance n-Type and Oxygen-Doped Ambipolar Field-Effect Transistors. Adv. Mater. 2013, 25(45), 6589).

An oxidant in the embodiment was duroquinone, coenzyme Q10 or ferric trichloride, which was purchased from Shanghai Bide Pharmaceutical Technology Co. Ltd. Embodiment 1: a method for preparing an n-type conjugated polymer blend

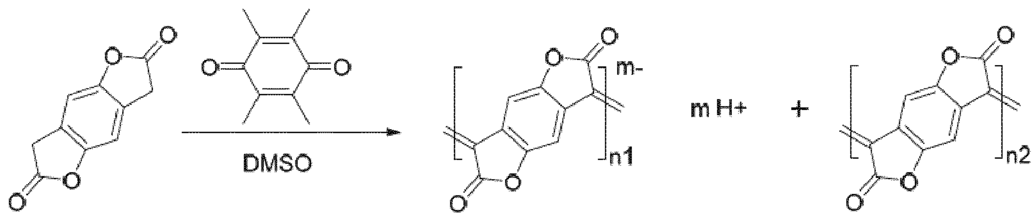


I

II

3,7-dihydrobenzo [1,2-b:4,5-b'] difuran-2,6-dione (1 mmol), and an oxidant of duroquinone (1.5 mmol), were dissolved in 2 ml of dimethyl sulfoxide (DMSO), degassed under vacuum, and protected by filling with nitrogen. The above mixture solution was stirred in a nitrogen atmosphere at a temperature of 100°C for 4 h. A resulting crude product was then diluted to approximately 10 mg/ml with DMSO and filtered using a polytetrafluoroethylene filter head with 0.45 μm of a pore size. A resulting filtrate was concentrated using a rotary evaporator and dialyzed in a DMSO solution for 7 days using a dialysis bag (a cut-off molecular weight = 10 kDa) to obtain the product of the n-type conjugated polymer blend. Where Formula I and Formula II could be interconverted by resonance as described above, and m/(n1+n2) was about 0.86. The test method for a ratio was obtained by fitting using an X-ray photoelectron energy spectrum (the same applies hereinafter). A test was performed by gel permeation chromatography with DMSO as a mobile phase. The molecular weight Mn=428.2 kDa and PDI=1.22.

Fig. 1 showed an absorption spectrogram of a product in Embodiment 1 in a thin film state with an absorption edge over 2400 nm, illustrating the presence of polarons in the polymer and proving the presence of negative charges in a mixture system that could move freely in a conjugated backbone. Fig. 2 showed a graph of a gel permeation chromatography test of a product in Embodiment 1, illustrating the production of the polymer and a supramolecular compound.

Fig. 3 showed the X-ray photoelectron energy spectrum of a product in Embodiment 1. Fig. 3 (a) showed an XPS spectrum of an element of oxygen (oxygen 1s peak) and Fig. 3 (b) showed a total spectrum. Therefore, a semi-quantitative ratio between different peaks of an element of oxygen in Fig. 3(a)-(b) could be used to calculate the relationship between m as well as n1 and n2. Therefore, with this method, the relative contents of Formula I and Formula

II can be determined.

Embodiment 2: a method for preparing an n-type conjugated polymer blend

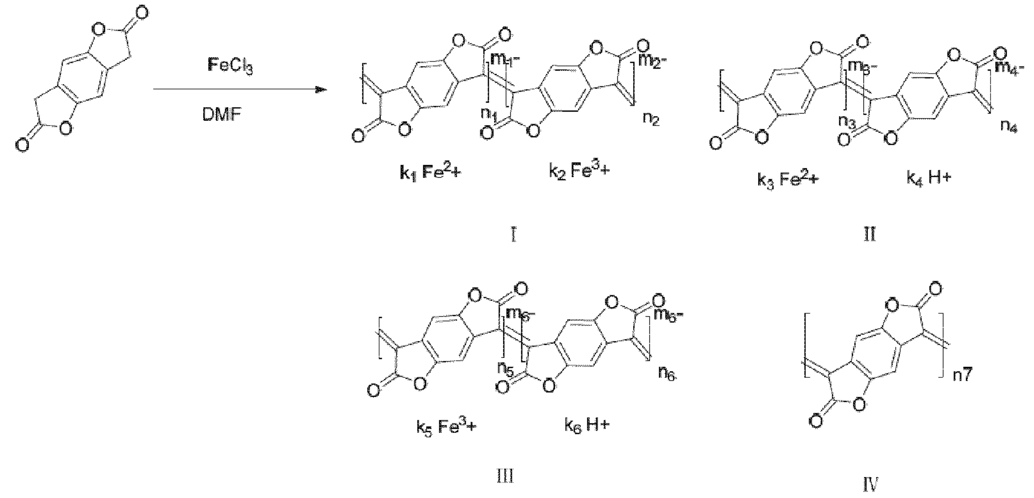


I

II

III

IV

3,7-dihydrobenzo [1,2-b:4,5-b'] difuran-2,6-dione (1 mmol), and an oxidant of ferric trichloride (2 mmol) were dissolved in 2 ml of N, N-dimethylformamide (DMF), degassed under vacuum, and protected by filling with nitrogen. The above mixture solution was stirred in a nitrogen atmosphere at a temperature of 100°C for 2 h. The resulting product of the n-type conjugated polymer blend was gradually precipitated out from a reaction system, cooled to a room temperature and then added with 50 mL of ethanol. After thorough stirring, the product was filtered and washed with deionized water, ethanol and tetrahydrofuran. The resulting product of the n-type conjugated polymer blend was a mixture of Formula (I), Formula (II), Formula (III) and Formula (IV). The resulting product was insoluble in an organic solvent due to coupling of a ferric ion. A solid was characterized.

Where, 2(k1+k3)+3(k2+k5)+(k4+k6)=m1+m2+m3+m4+m5+m6.
and (k4+k6):(k1+k3):(k2+k5) was approximately 8:1:0.2.
(m1+m2+m3+m4+m5+m6):(n1+n2+n3+n4+n5+n6+n7) was about 0.9.

Embodiment 3: a method for preparing an n-type conjugated polymer blend

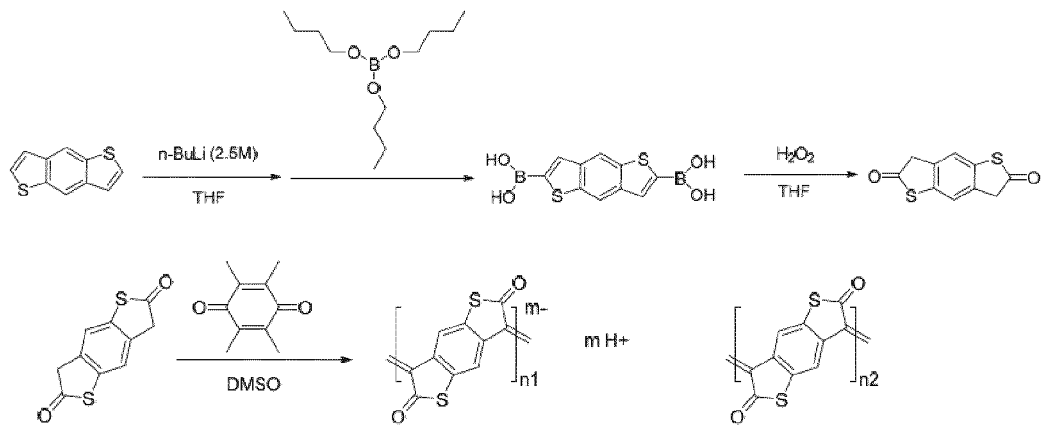


(1) Benzodithiophene (23 mmol) was dissolved in anhydrous tetrahydrofuran (300 ml), then cooled down to a temperature of -78°C, slowly added into n-butyllithium (2.5 M of a hexane solution, 25.8 ml, 64.4 mmol) within 90 min, stirred for 3 h at a temperature of -78°C, then warmed up to 0°C, added with tri-n-butyl borate (60 mmol), stirred for 1 h, then gradually warmed up to the room temperature of 25°C and continuously stirred for 8 h. A solution was

concentrated to 200 ml using a rotary evaporator, added with 200 ml of 0.5 M of hydrochloric acid and filtered to obtain a crude product. A product was precipitated out from the crude product using tetrahydrofuran/hexane, washed with ice-toluene and dried to obtain a solid (yield = 61%) for a next step of reaction.

(2) The solid (7.5 mmol) from the previous step was dissolved in tetrahydrofuran (100 ml), added with 2.5 ml of an aqueous hydrogen peroxide solution (30 wt%) at a temperature of 0°C, and stirred for 6 h at a room temperature. After the solvent was removed using the rotary evaporator, the product was purified using a silica gel packed chromatographic column (200-300 mesh) with ethyl acetate: petroleum ether = 4:1 as an eluent. An obtained solid was washed with hexane and methanol to obtain 3,7-dihydrobenzo[1,2-b:4,5-b'] dithiophene-2,6-dione (yield = 53%). $^1$HNMR($CDCl_3$, 500MHz) δppm : 7.31 (s, 2H), 3.98 (s, 4H).

(3) 3,7-dihydrobenzo [1,2-b:4,5-b'] dithiophene-2,6-dione (1 mmol), and duroquinone (2 mmol) were dissolved in 2 ml of DMSO, degassed under vacuum and protected by filling with nitrogen. The solution was stirred for 10 h under the nitrogen atmosphere at a temperature of 120°C, diluted to about 10 mg/ml and filtered using a 0.45 μm pore size of a polytetrafluoroethylene filter head. The solution was concentrated using the rotary evaporator and dialyzed in the DMSO solution using a dialysis bag (a cut-off molecular weight = 10 kDa) for 3 days to obtain the product of the n-type conjugated polymer blend. The resulting product of the n-type conjugated polymer blend was a mixture of Formula (I) and Formula (II). Formula I and Formula II could be interconverted by resonance as described above, and m/(n1+n2) was about 0.42. A test was performed by gel permeation chromatography with DMSO as a mobile phase. The molecular weight Mn=12 kDa and PDI=1.31.

Embodiment 4: a method for preparing an n-type conjugated polymer blend

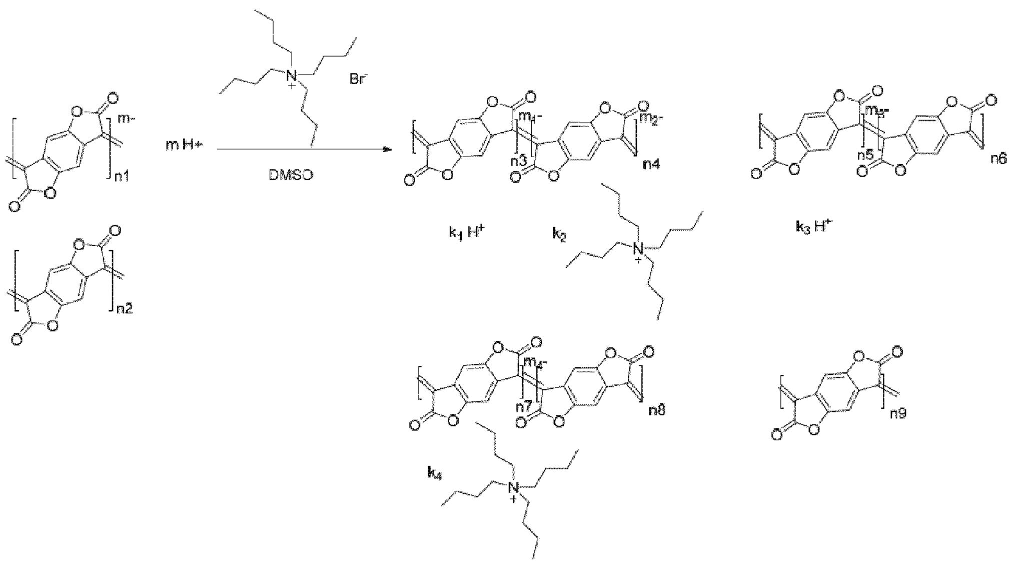


20 mL of a DMSO solution of the product obtained in Embodiment 1 (a concentration of 15 mg/mL) was diluted with 20 mL of DMSO, added with 2 g of tetrabutylammonium bromide, and stirred at a room temperature for 14 days. A part of an ion exchange product was gradually precipitated out during a reaction. 50 mL of ethanol was added to a reaction system, which was stirred thoroughly and filtered. A resulting powder was washed with the ethanol and tetrahydrofuran.

Where k1+k2+k3+k4=m1+m2+m3+m4=m.

n3 +n4+n5+n6+n7+n8+n9=n1+n2.

(k2+k4)/(k1+k3) was about 0.2.

Embodiment 5: a method for preparing an n-type conjugated polymer blend

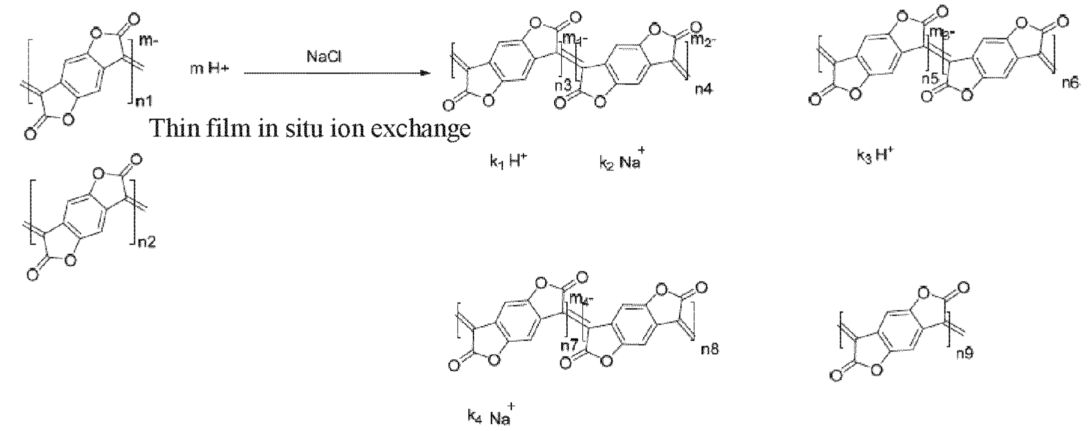


A DMSO solution of 40 μL of a product obtained in Embodiment 1 (concentration of 15 mg/mL) was added dropwise onto a 10 mm × 10 mm glass substrate, and dried to obtain a film. The film was immersed in an aqueous solution of saturated NaCl for 12 h, then washed with deionized water and dried to obtain a film of ion exchange product of the n-type conjugated polymer blend, which was directly performed with subsequent characterization.

Where k1+k2+k3+k4=m1+m2+m3+m4=m.

n3 +n4+n5+n6+n7+n8+n9=n1+n2.

(k2+k4)/(k1+k3) was about 0.04.

Embodiment 6: a method for preparing an n-type conjugated polymer blend

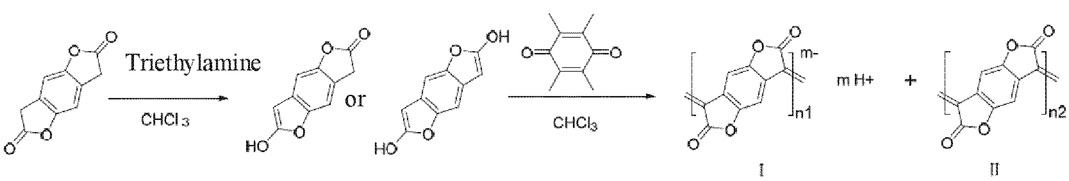


Under the protection of nitrogen, 3,7-dihydrobenzo [1,2-b:4,5-b'] difuran-2,6-dione (1 mmol) was dissolved in 5 mL of trichloromethane, added with 2 mmol of triethylamine, and stirred at a room temperature. After the solution turned dark green, an oxidant of duroquinone (1.5 mmol) was added. The above mixture solution was stirred in a nitrogen atmosphere at a temperature of 100°C for 24 h and filtered to obtain a crude product. A resulting crude product was then washed with tetrahydrofuran, dissolved in 20 mL of DMSO, and filtered using a 0.45 μm pore size of a polytetrafluoroethylene filter head. A resulting filtrate was dialyzed in a DMSO solution using a dialysis bag (a cut-off molecular weight = 10 kDa) for 7 days to obtain the product of the n-type conjugated polymer blend. Formula I and Formula II could be interconverted by resonance as described above, and m/(n1+n2) was about 0.62. A test was performed by gel permeation chromatography with DMSO as a mobile phase. The molecular weight Mn=1 1 kDa and PDI=1.72.

Test Example 1

With a DMSO solution of an n-type conjugated polymer blend obtained in Embodiment 1, a film was prepared on a glass substrate using a spin-coating film formation method. After the film was dried under vacuum, the conductivity of the film was measured using a four-foot probe method. Fig. 4 showed a schematic diagram of a four-probe conductivity test of a product of Embodiment 1. The specific steps were as follows:
A quartz glass sheet was washed with acetone, a micron-level semiconductor special detergent, deionized water and isopropyl alcohol as a cleaning solvent in ultrasonic cleaner in turn. The surface of the quartz glass sheet was dried with nitrogen gas after washing, and was dried with an infrared lamp. The quartz glass sheet was then placed in a constant temperature oven for backup. Before use, the glass sheet was bombarded with plasma in a plasma etcher for 10 min.

After the preparation of the glass sheet was finished, the glass sheet was placed on a heating table, heated at a temperature of 110°C and transferred to a rotary gelatinometer (KW-4A). The product of Embodiment 1 prepared above was spin-coated at a high speed (the mass concentration of the conjugated polymer solution was 15 mg/ml), and the thickness of a monitored film was simultaneously measured realistically with a step meter. After film formation was

completed, voltage and current curves were tested using a four-foot probe conductivity tester. A test schematic diagram was shown in Fig. 4. A voltage V and a current I were shown in Fig. 5(a)-(b). The conductivity $\sigma$ was calculated using a film thickness d and a correction factor, with a calculated formula as:

$$\sigma = I/(C \times V \times d) \text{ (where C was determined by both built-in probe calibration parameters of an instrument and a specimen size).}$$

Powder of products obtained in Embodiment 2 and Embodiment 4 was prepared as a thin film with a diameter of 15 mm and a thickness of 1 mm using a press method. The conductivity of the film was measured using the four-foot probe method.

The conductivity of the film of the product obtained after ion exchange in Embodiment 5 was directly measured using a four-foot probe method with the following results.

Table 1: Conductivity Tests of Products of Embodiment 1, Embodiment 2, Embodiment 4 and Embodiment 5

| n-type Conjugated Polymer Blend | Conductivity (S/cm) |
|---|---|
| Embodiment 1 | 2150 |
| Embodiment 2 | 942 |
| Embodiment 4 | 1720 |
| Embodiment 5 | 2408 |

As can be seen from Table 1, the n-type conjugated polymer blend prepared by the present invention had high conductivity. The regulation of the conductivity could be achieved by different counterions.

Here, the n-type conjugated polymer blend of Embodiment 1 was illustrated as follows: the n-type conjugated polymer blend prepared by the present invention could resonate hydrogen protons and negative charges on a backbone. In addition, in a solid state, due to the formation of a supramolecular interaction, the hydrogen protons were bound between molecules, thus weakening binding to the negative charges, allowing the negative charges to move freely on a conjugated backbone and achieving high n-type conductivity.

Test Example 2

A thermoelectric test was performed on an n-type conjugated polymer blend obtained in an embodiment.

The thermoelectric properties of a material were commonly described by the thermoelectric figure of merit (ZT), which was given by the following formula:

$$ZT=S^2\sigma T/\kappa;$$

Where S represents a Seebeck coefficient. $\sigma$ represents conductivity. $\kappa$ represents thermal conductivity, and T represents a temperature at which a device operates. The thermal conductivity of an organic material was much lower than that of an inorganic material, so a power factor (PF) was commonly used to describe the thermoelectric properties of the organic material, where $PF=S^2\sigma$.

In this test example, taking as an example the n-type conjugated polymer blend prepared above, a sample preparation process was the same as the one used in Test Example 1 to test the Seebeck coefficient and characterize thermoelectric properties.

The two ends of a device were placed in a temperature gradient field. Fig. 5(a) showed a diagram of a trend of the thermal voltage difference of a product in Embodiment 2, and Fig. 5(b) showed a fitted diagram of a trend of the thermal voltage difference of a product in Embodiment 2 with temperature. A corresponding temperature difference electric potential was tested with the change in temperature difference between the two ends of the device to measure the Seebeck coefficient. As seen on Figs. 5(a)-(b), the polymer of the embodiment of the present invention exhibited the Seebeck effect of the product at a weak temperature difference and generated the temperature difference electric potential of more than 30 $\mu$V/K.

Fig. 6 showed the conductivity-Seebeck coefficient-power factor performance graph for the product of Embodiment 1. The power factor was obtained from $PF=S^2\sigma$. From the above tests, it can be obtained that the power factor

was close to 100$\mu Wm^{-1}K^{-2}$ at a room temperature, and was at a higher level among the n-type conjugated polymer in the prior art.

Relevant data were shown in Table 2.

Table 2. Seebeck Coefficients of n-type Conjugated Polymer Blends of Embodiment 1, Embodiment 2 and Embodiment 4

| n-type Conjugated Polymer Blend | Seebeck Coefficient ($\mu V/K$) | Power Factor ($\mu Wm^{-1}K^{-2}$) |
|---|---|---|
| Embodiment 1 | -19.09 | 92.9 |
| Embodiment 2 | -28.67 | 77.4 |
| Embodiment 4 | -23.59 | 95.7 |

Test Example 3

The n-type conjugated polymer blend obtained in Embodiment 1 was tested for an organic electrochemical transistor device. The organic electrochemical transistor device was shown in Fig. 7.

The manufacturing process of the above organic electrochemical transistor was as follows: First, a glass base was washed with acetone and isopropyl alcohol in turn, and then the surface of the glass base was blown with nitrogen gas to be dried. A layer of Cr/Au (10 nm/100 nm) substrate was deposited on a glass substrate using a sputtering method to build a source electrode and a drain electrode. The surface of the substrate was passivated and spin-coated with a DMSO solution of the n-type conjugated polymer in Embodiment 1 as an active layer material in the organic electrochemical transistor, and baked at a temperature of 100°C for 10 min under nitrogen, with a film thickness of about 70 nm and a trench area of 1 mm × 1mm. The performance of the organic electrochemical transistor was characterized in air using an Ag/AgCl electrode as a gate electrode, a 0.1 M NaCl solution as an electrolyte, and Keithley 4200 to record a voltage Vgs between the source electrode and the gate electrode and a current Ids between the drain electrode and the gate electrode.

The tested performance of the above organic electrochemical transistor device was shown in Fig. 8. The organic electrochemical transistor device based on the n-type conjugated polymer blend had a good electrochemical response with a transconductivity close to 11 mS, and had important application prospects in the field of organic biosensing.

The above test examples illustrated that the n-type conjugated polymer blend of the present invention had a wide range of promising applications as a highly conductive material in an organic optoelectronic device.

It is obvious to a person skilled in the art that the present invention not limited to the details of the exemplary embodiments described above, and that the present invention can be realized in other specific forms without departing from the spirit or basic features of the present invention. Therefore, the embodiments should be regarded as exemplary and non-limiting from any standpoint. The scope of the present invention is defined by the appended claims rather than the above description. It is therefore intended that all variations falling within the meaning and scope of equivalent elements of claims be encompassed by the present invention.

In addition, it should be understood that although this specification is described in terms of embodiments, not each embodiment only includes an independent technical solution. This description in the specification is only for the sake of clarity. A person skilled in the art should take the specification as a whole. The technical solutions in each embodiment can also be appropriately combined to form other implementations that can be understood by a person skilled in the art.

## Claims

**1.** An n-type conjugated polymer blend, comprising an n-type conjugated polymer containing a counterion, and/or an n-type conjugated polymer,
wherein,

the n-type conjugated polymer containing the counterion comprises one or more polymerization units 1,
the polymerization unit 1 has the following structure (I):

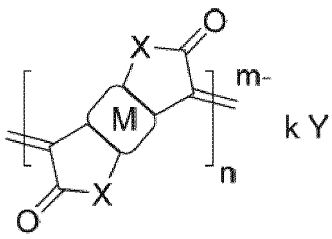

(I);

the n-type conjugated polymer comprises one or more polymerization units 2, the polymerization unit 2 has the following structure (II):

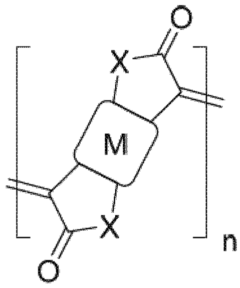

(II);

in each of the polymerization unit 1 or the polymerization unit 2,
X is independently selected from O, S, Se, Te or N-$R_1$;
the $R_1$ is selected from one or more of a hydrogen atom, alkyl, alkylidene, an alkyl derivative, and an alkylidene derivative;
one or more carbons on the alkyl derivative or the alkylidene derivative are substituted with one or more of an oxygen atom, amino, sulfonyl, carbonyl, aryl, alkenyl, alkynyl, ester, cyano, and nitro.
and/or
one or more hydrogens on the alkyl derivative or alkylidene derivative are substituted with one or more of halogen, hydroxyl, the amino, carboxyl, the cyano, the nitro, the aryl, alkylene, and alkyne;
where m, n and k are positive integers;
the M is a conjugated portion of a structure of the n-type conjugated polymer containing the counterion or the n-type conjugated polymer, and a structure of the M is selected from one of aromatic ring, aromatic heterocycle, fused aromatic ring, fused aromatic heterocycle;
the Y is a counterion in the structure of the n-type conjugated polymer containing the counterion or the n-type conjugated polymer, and the counterion is selected from one of an organic cation or an inorganic cation;
the n-type conjugated polymer blend is prepared from a raw material (III), and the raw material (III) has the following structure:

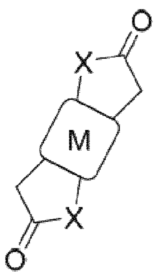

and/or an enol-transformed form of the raw material; the enol-transformed form is

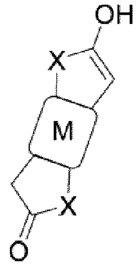

and/or

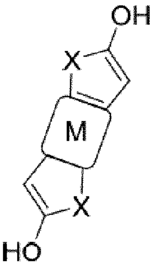

.

2. The n-type conjugated polymer blend according to Claim 1, wherein a structure of a conjugated portion M of the structure of the n-type conjugated polymer containing the counterion or the n-type conjugated polymer is independently selected from the following structures of

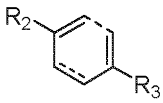 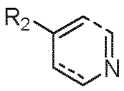 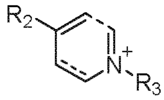 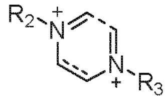

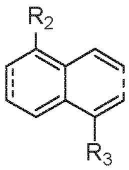 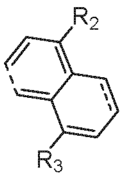 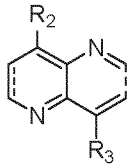 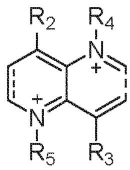

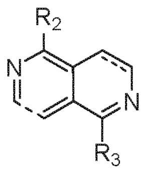 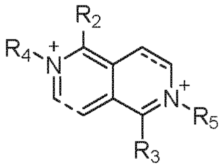 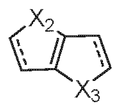 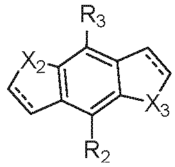

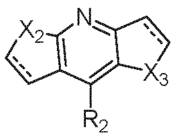 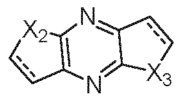 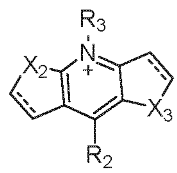 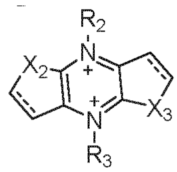

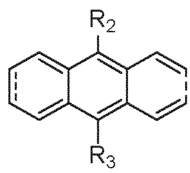 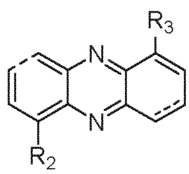 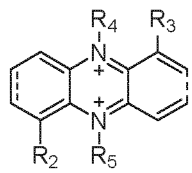 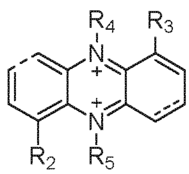

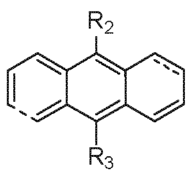 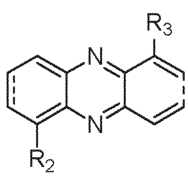 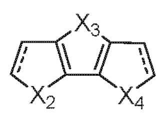 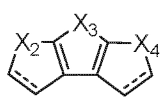

;

wherein the $X_2$-$X_4$ are independently selected from O, S, Se, Te or N-$R_6$;
$R_2$-$R_6$ is independently selected from one or more of the hydrogen atom, the hydroxyl, the nitro, the halogen, the cyano, the nitro, the alkyl, the alkyl derivative, the alkylidene, and the alkylidene derivative;

one or more carbons on the alkyl derivative or the alkylidene derivative are substituted with one or more of the oxygen atom, the amino, the sulfonyl, the carbonyl, the aryl, the alkenyl, the alkynyl, the ester, the cyano, and the nitro;

and/or

one or more hydrogens on the alkyl derivative or the alkylidene derivative are substituted with one or more of the halogen, the hydroxyl, the amino, the carboxyl, the cyano, the nitro, the aryl, the alkylene, and the alkyne.

**3.** The n-type conjugated polymer blend according to Claim 1, wherein a structure of a counterion Y of the structure of the n-type conjugated polymer containing the counterion is selected from one of the following structures:

$H^+$ 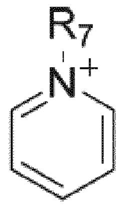 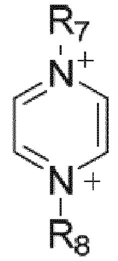 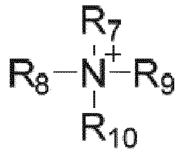 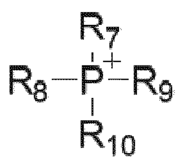

$Na^+$, $K^+$, $Mg^{2+}$, $Ca^{2+}$, $Ba^{2+}$, $A1^{3+}$, $Fe^{2+}$, $Fe^{3+}$, $Zn^{2+}$, $Cu^{2+}$ and $Ag^+$;

or is selected from one of an amine-salt-type cationic surfactant, a quaternary-ammonium-salt-type cationic surfactant, a heterocycle-type cationic surfactant, a sulfonium salt, iodine, and other salt compounds.

**4.** The n-type conjugated polymer blend according to Claim 1, wherein

(1) when the n-type conjugated polymer containing the counterion is a homopolymer, the structure of the n-type conjugated polymer containing the counterion is selected from one of the following structures:

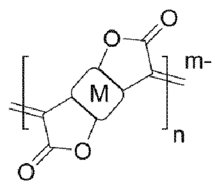 k Y 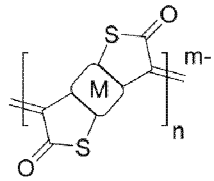 k Y 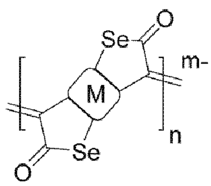 k Y

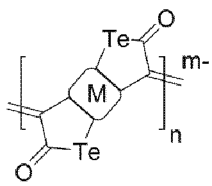 k Y 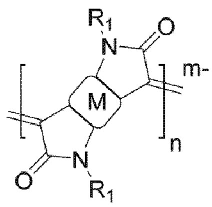 k Y ;

or

(2) when the n-type conjugated polymer containing the counterion is a copolymer,

the structure of each of the polymerization unit 1 of the n-type conjugated polymer containing the counterion is independently selected from the following structures:

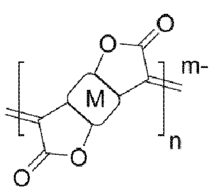 k Y 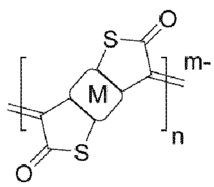 k Y 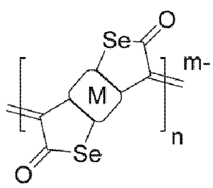 k Y

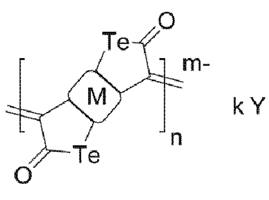
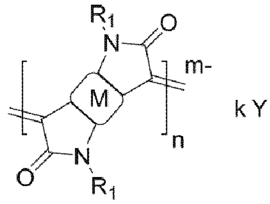

;

wherein,

the $R_1$ is selected from one or more of a hydrogen atom, alkyl, alkylidene, an alkyl derivative, and an alkylidene derivative;
one or more carbons on the alkyl derivative or the alkylidene derivative are substituted with one or more of the oxygen atom, the amino, the sulfonyl, the carbonyl, the aryl, the alkenyl, the alkynyl, the ester, the cyano, and the nitro;
and/or
one or more hydrogens on the alkyl derivative or alkylidene derivative are substituted with one or more of the halogen, the hydroxyl, the amino, the carboxyl, the cyano, the nitro, the aryl, the alkylene, and the alkyne;
where m, n and k are positive integers;
the M is the conjugated portion of the structure of the n-type conjugated polymer containing the counterion, and the structure of the M is selected from one of an the aromatic ring, the aromatic heterocycle, the fused aromatic ring, the fused aromatic heterocycle;
the Y is the counterion portion of the n-type conjugated polymer containing the counterion, and the counterion is selected from one of an organic cation and an inorganic cation.

**5.** The n-type conjugated polymer blend according to Claim 1, wherein

(1) when the n-type conjugated polymer is the homopolymer, the structure of the n-type conjugated polymer is selected from one of the following structures:

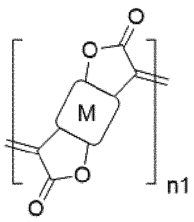
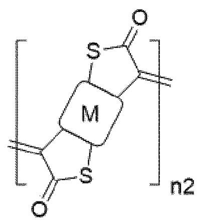
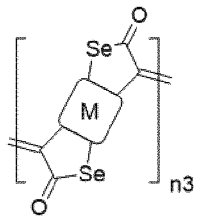

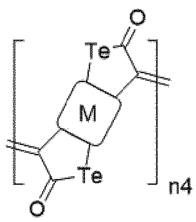
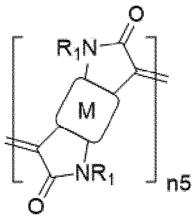

;

(2) when the n-type conjugated polymer is the copolymer,

a structure of each of the polymerization unit 2 of the n-type conjugated polymer is independently selected from the following structures:

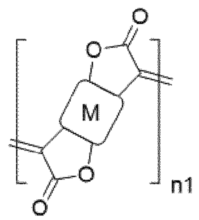 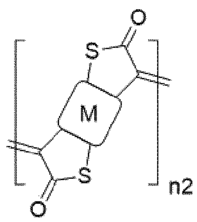 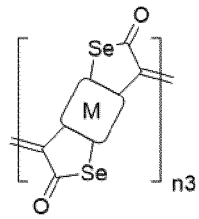

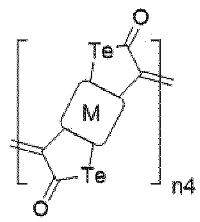 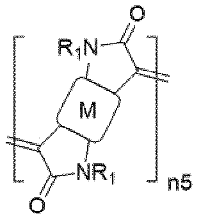

;

the n1-n5 are independently positive integers.

**6.** The n-type conjugated polymer blend according to Claim 1, wherein a dynamic interconversion is provided between the n-type conjugated polymer containing the counterion and the n-type conjugated polymer.

**7.** The n-type conjugated polymer blend according to Claim 6, wherein a ratio of an amount of substance of the n-type conjugated polymer containing the counterion to an amount of substance of the n-type conjugated polymer is 0.1-10.

**8.** A method for preparing the n-type conjugated polymer blend according to Claim 1, wherein

(1) when the n-type conjugated polymer containing a counterion and/or the n-type conjugated polymer is a homopolymer, the method of preparing the n-type conjugated polymer blend comprises the following steps of: mixing a raw material (III) and an oxidant and/or an ion exchange adjuvant in a solvent and heating to obtain the homopolymer;
or
(2) when the n-type conjugated polymer containing the counterion and/or the n-type conjugated polymer is a copolymer, the method of preparing the n-type conjugated polymer blend comprises the following steps of: mixing two or more of the raw materials (III), and the oxidant and/or the ion exchange adjuvant in the solvent and heating to obtain the copolymer.

**9.** The method for preparing the n-type conjugated polymer blend according to Claim 8, wherein the oxidant is selected from one or more of an organic type oxidant and an inorganic type oxidant.

**10.** The method for preparing the n-type conjugated polymer blend according to Claim 8, wherein the oxidant is selected from one or more of oxygen, peroxide, metal halide, persulfate, perborate, subhaloate, halogenite, a quinones compound, and a benzylhydroperoxide compound.

**11.** The method for preparing the n-type conjugated polymer blend according to Claim 8, wherein the ion exchange adjuvant is selected from one or more of an inorganic salt containing a cation, and an organic salt containing a cation.

**12.** The method for preparing the n-type conjugated polymer blend according to Claim 8, wherein the solvent is selected from a solvent 1 or a solvent 2, or a mixture of the solvent 1 and the solvent 2.

the solvent 1 is selected from one or more of water, a nitrile solvent, an aromatic solvent, an alicyclic hydrocarbon solvent, an alicyclic hydrocarbon solvent, a halogenated hydrocarbon solvent, an alcohol solvent, an ether solvent, an ester solvent, a sulfone solvent, a ketone solvent, and an amide solvent;
the solvent 2 is a deuterated solvent of the solvent 1.

**13.** Application of the n-type conjugated polymer blend of Claim 1 in an organic optoelectronic device.

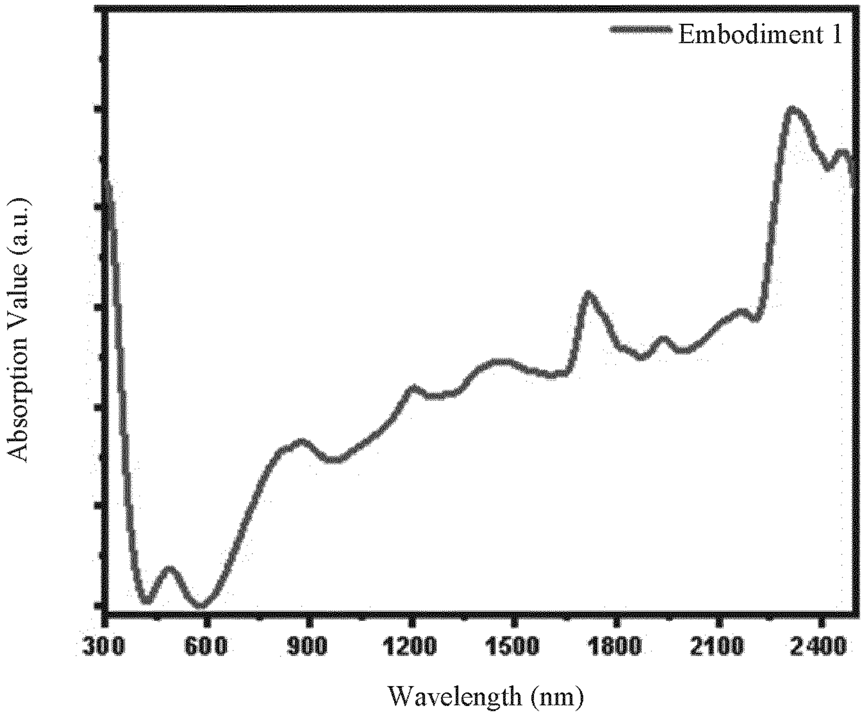
Embodiment 1
Absorption Value (a.u.)
300
600
900
1200
1500
1800
2100
2400
Wavelength (nm)

Fig. 1

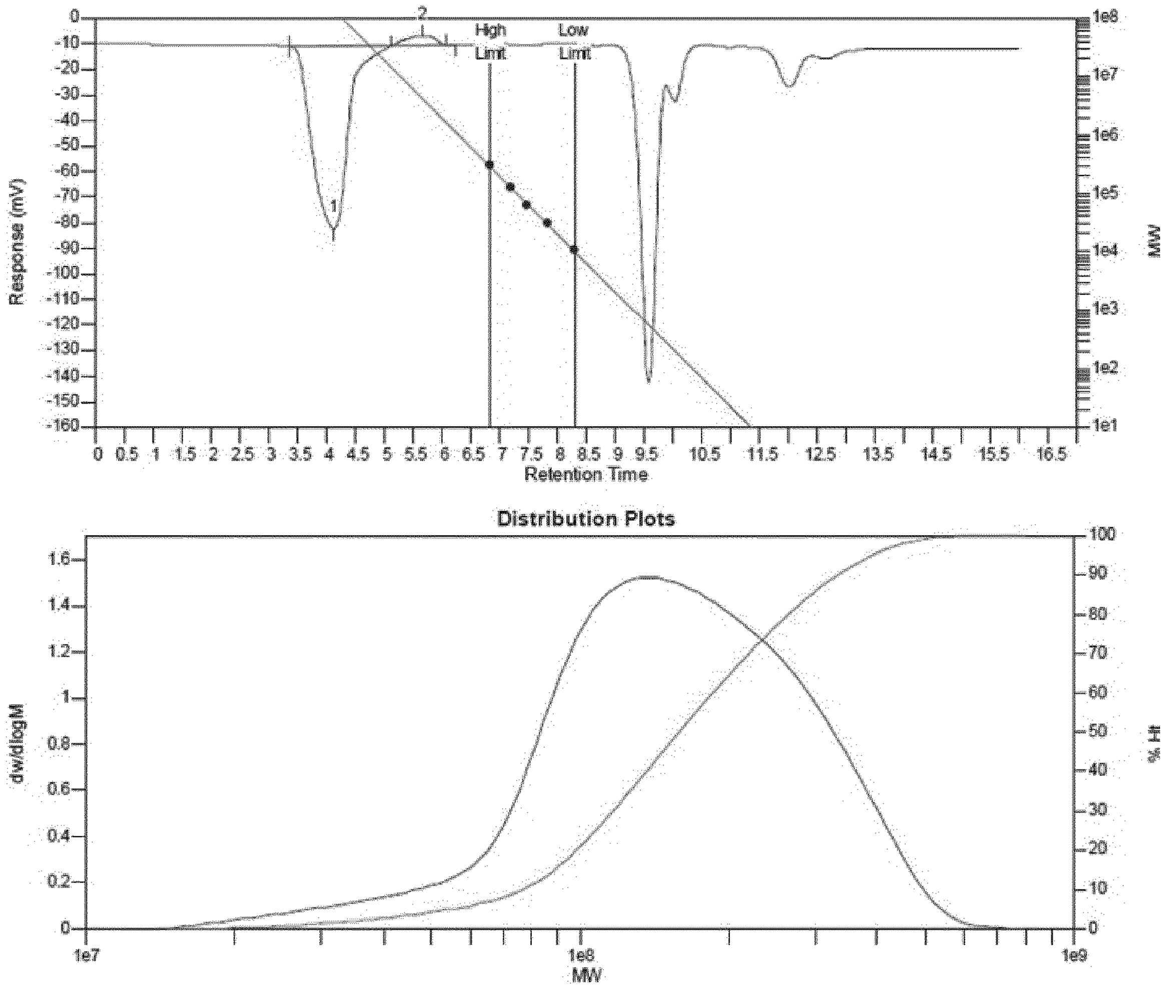


**MW Averages**

| Peak No | Mp | Mn | Mw | Mz | Mz+1 | Mv | PD |
|---|---|---|---|---|---|---|---|
| 1 | 138485175 | 125561026 | 180237109 | 237374176 | 292756158 | 172126593 | 1.43545 |
| 2 | 4214935 | 4282082 | 5240601 | 6468368 | 7689783 | 5065552 | 1.22384 |

Fig. 2

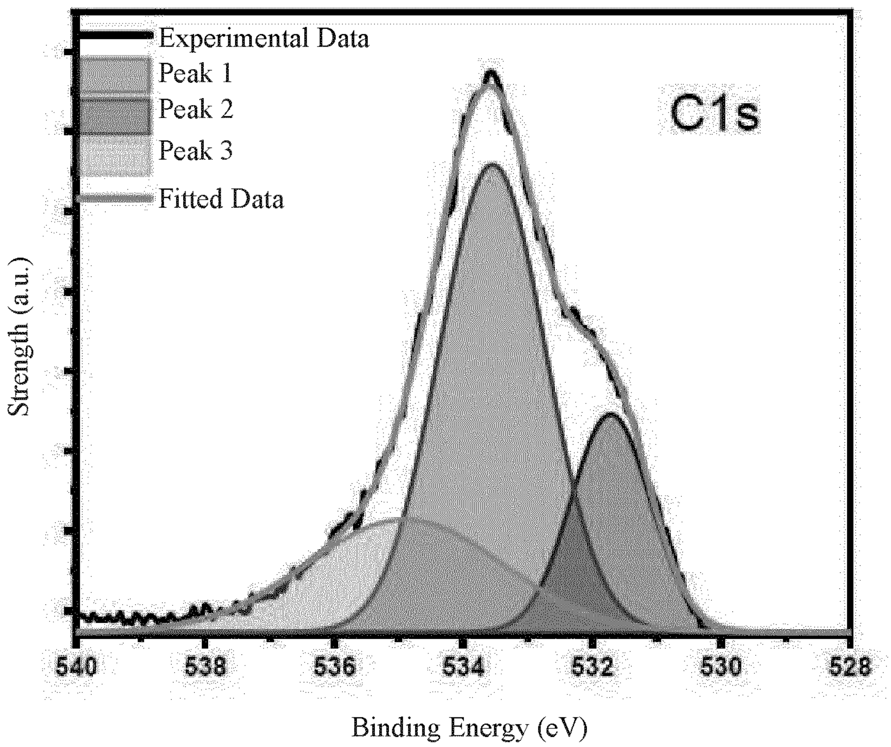
Experimental Data
Peak 1
Peak 2
Peak 3
Fitted Data
C1s
Strength (a.u.)
540
538
536
534
532
530
528
Binding Energy (eV)

(a)

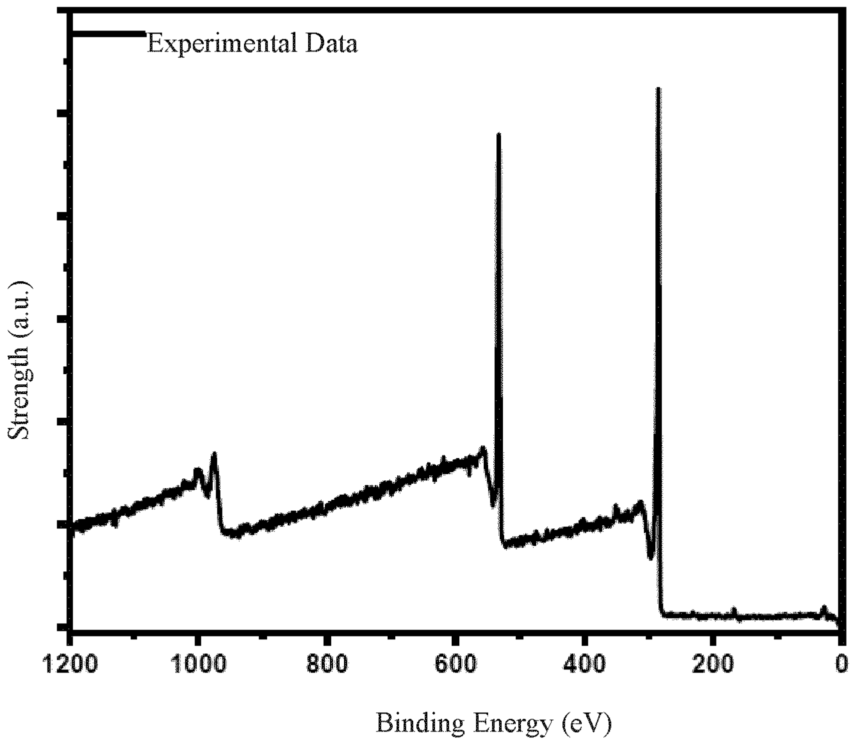
Experimental Data
Strength (a.u.)
1200
1000
800
600
400
200
0
Binding Energy (eV)

(b)

Fig. 3

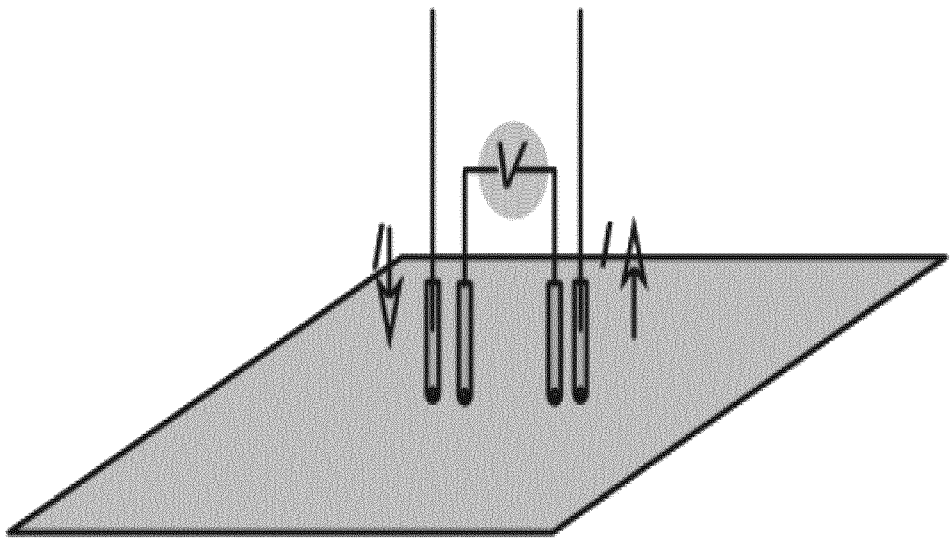


Fig. 4

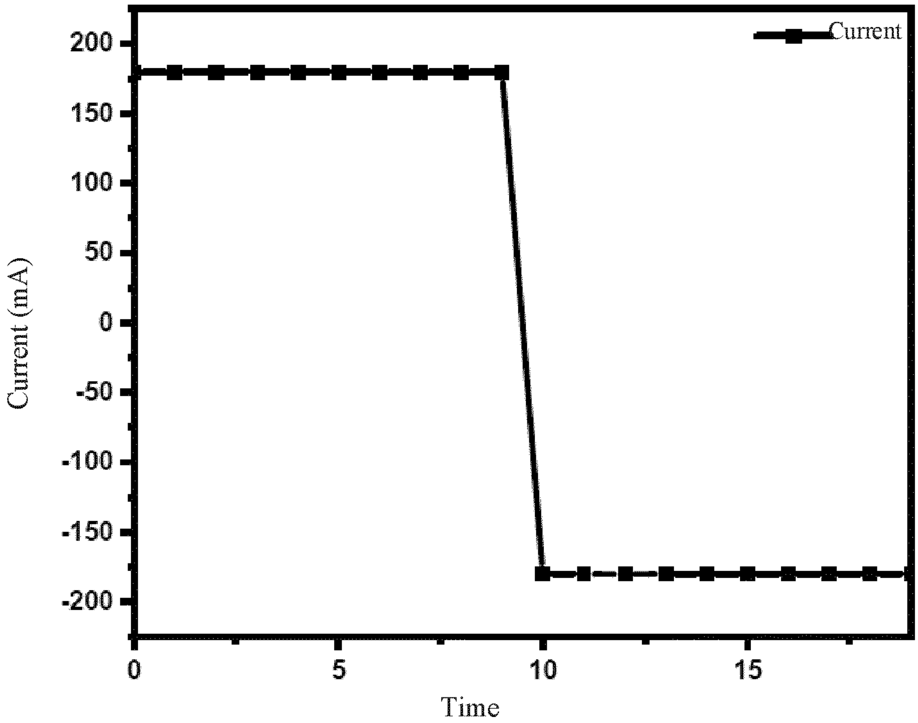
Current
200
150
100
50
0
-50
-100
-150
-200
Current (mA)
0
5
10
15
Time

(a)

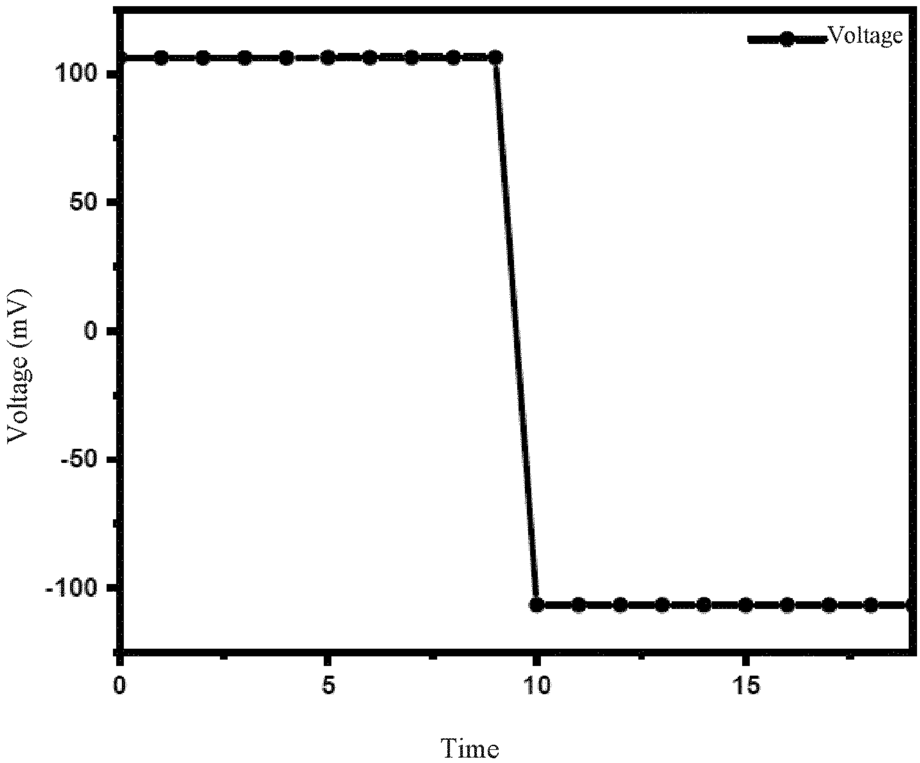
Voltage
100
50
0
-50
-100
Voltage (mV)
0
5
10
15
Time

(b)

Fig. 5

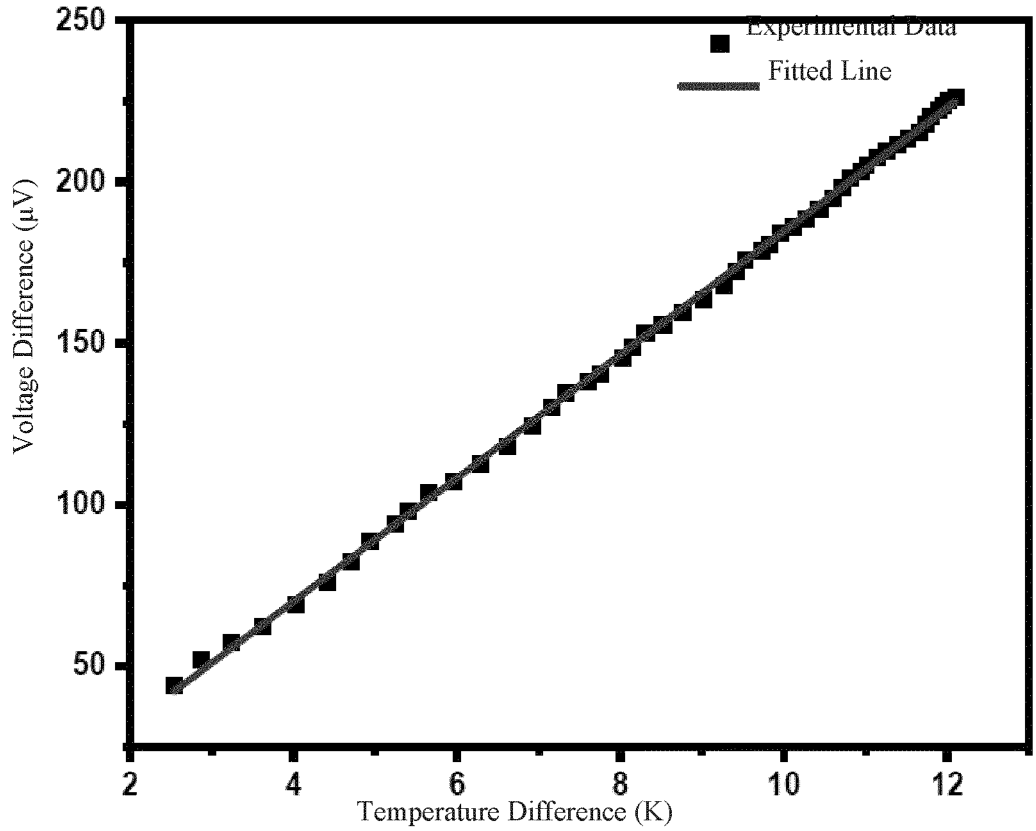
250
200
150
100
50
2
4
6
8
10
12
Voltage Difference (μV)
Temperature Difference (K)
Experimental Data
Fitted Line

Fig. 6

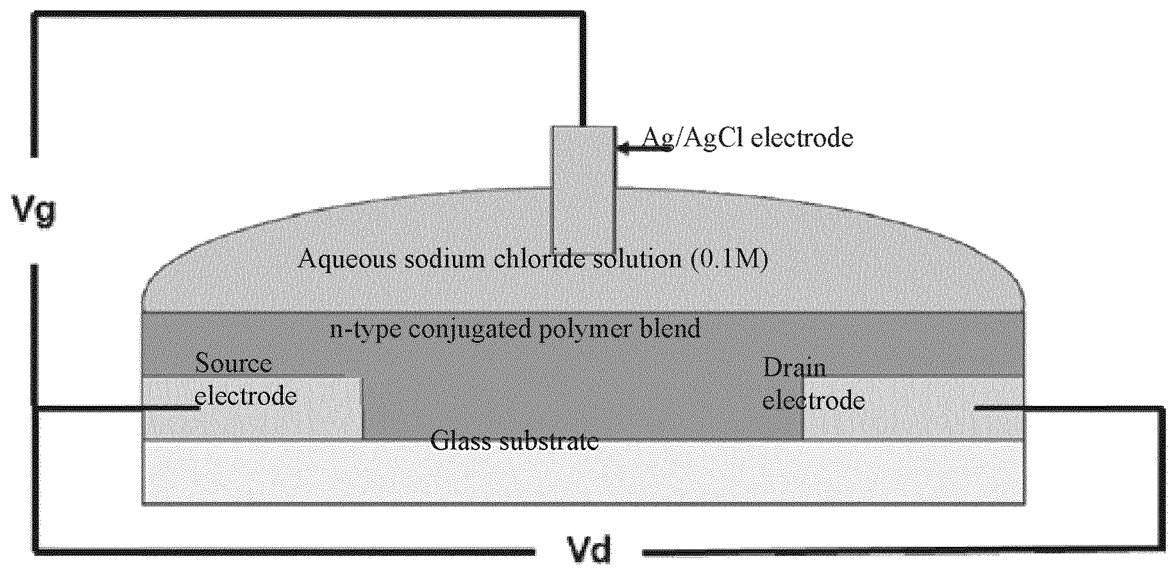
Ag/AgCl electrode
Vg
Aqueous sodium chloride solution (0.1M)
n-type conjugated polymer blend
Source electrode
Drain electrode
Glass substrate
Vd

Fig. 7

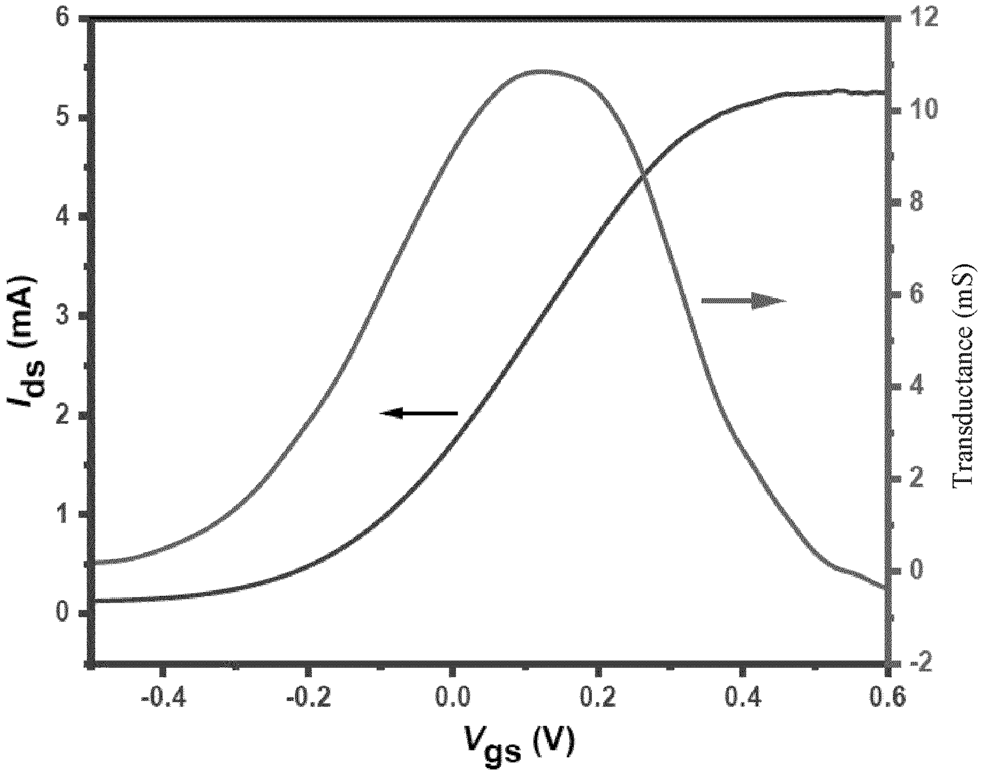
6
5
4
3
2
1
0
$I_{ds}$ (mA)
12
10
8
6
4
2
0
-2
Transductance (mS)
-0.4
-0.2
0.0
0.2
0.4
0.6
$V_{gs}$ (V)

Fig. 8

## INTERNATIONAL SEARCH REPORT

International application No.
**PCT/CN2021/124545**

**A. CLASSIFICATION OF SUBJECT MATTER**

C08G 61/12(2006.01)i

According to International Patent Classification (IPC) or to both national classification and IPC

**B. FIELDS SEARCHED**

Minimum documentation searched (classification system followed by classification symbols)

C08G

Documentation searched other than minimum documentation to the extent that such documents are included in the fields searched

Electronic data base consulted during the international search (name of data base and, where practicable, search terms used)

EPODOC, WPI, WebofScience, STN, CNPAT, CNKI: n?type, conjugated, +dione, benzo+furan, benzo+thiophene, duoquinone, ferric w chloride, coenzyme, n型共轭, 苯并+呋喃, 苯并+噻吩, 杜醌, 三氯化铁, 辅酶.

**C. DOCUMENTS CONSIDERED TO BE RELEVANT**

| Category* | Citation of document, with indication, where appropriate, of the relevant passages | Relevant to claim No. |
|---|---|---|
| A | CN 110862517 A (PEKING UNIVERSITY) 06 March 2020 (2020-03-06) claims 1-12 | 1-13 |
| A | CN 106977701 A (SOUTH CHINA UNIVERSITY OF TECHNOLOGY) 25 July 2017 (2017-07-25) description, paragraphs 009-0031 | 1-13 |
| A | CN 103804271 A (PEKING UNIVERSITY) 21 May 2014 (2014-05-21) claims 1-11 | 1-13 |
| A | WO 2017148864 A1 (BASF SE.) 08 September 2017 (2017-09-08) claims 1-20 | 1-13 |
| A | US 8859718 B2 (PLEXTRONICS INC. et al.) 14 October 2014 (2014-10-14) claim 1, and embodiments 1-9 | 1-13 |
| A | EP 0328983 A1 (HOECHST AKTIENGESELLSCHAFT) 23 August 1989 (1989-08-23) embodiments 1-9 | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 June 2022** | **24 June 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/124545**

| Patent document cited in search report | Publication date (day/month/year) | Patent family member(s) | Publication date (day/month/year) |
|---|---|---|---|
| CN 110862517 A | 06 March 2020 | None | |
| CN 106977701 A | 25 July 2017 | None | |
| CN 103804271 A | 21 May 2014 | CN 103804271 B | 10 August 2016 |
| WO 2017148864 A1 | 08 September 2017 | EP 3423455 A1 | 09 January 2019 |
| | | US 2019048015 A1 | 14 February 2019 |
| | | KR 20180113610 A | 16 October 2018 |
| | | CN 108699073 A | 23 October 2018 |
| | | TW 201800412 A | 01 January 2018 |
| | | JP 2019508556 A | 28 March 2019 |
| US 8859718 B2 | 14 October 2014 | WO 2013059712 A1 | 25 April 2013 |
| | | US 2018228465 A1 | 16 August 2018 |
| | | JP 2014530940 A | 20 November 2014 |
| | | WO 2013059714 A1 | 25 April 2013 |
| | | US 2015105534 A1 | 16 April 2015 |
| | | KR 20140093679 A | 28 July 2014 |
| | | CN 103987735 A | 13 August 2014 |
| | | US 2013109813 A1 | 02 May 2013 |
| | | US 2013102889 A1 | 25 April 2013 |
| | | TW 201326251 A | 01 July 2013 |
| | | EP 2768868 A1 | 27 August 2014 |
| EP 0328983 A1 | 23 August 1989 | CN 1036019 A | 04 October 1989 |
| | | AU 2978789 A | 17 August 1989 |
| | | IL 89245 D0 | 10 September 1989 |
| | | PT 89689 A | 04 October 1989 |
| | | FI 890623 A0 | 09 February 1989 |
| | | JP H01261471 A | 18 October 1989 |
| | | DK 62489 D0 | 10 February 1989 |
| | | DE 3804523 A1 | 24 August 1989 |
| | | NO 890586 D0 | 10 February 1989 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Toward High Performance n-Type Thermoelectric Materials by Rational Modification of BDPPV Backbones. *J. Am. Chem. Soc.,* 2015, vol. 137, 6979
- High Conductivity and Electron-Transfer Validation in an n-Type Fluoride-Anion-Doped Polymer for Thermoelectrics. *Air. Adv. Mater.,* 2017, vol. 29, 1606928
- Rigid Coplanar Polymers for Stable n-Type Polymer Thermoelectrics. *Angew. Chem. Int. Ed.,* 2019, vol. 58, 11390
- A thermally activated and highly miscible dopant for n-type organic thermoelectrics. *Nat. Commun.,* 2020, vol. 11, 3292
- *Adv. Mater.,* 2021, vol. 33, 200546